(19) Europäisches Patentamt
European Patent Office
Office européen des brevets

(11) Veröffentlichungsnummer : **0 008 111**
**B1**

(12) # EUROPÄISCHE PATENTSCHRIFT

(45) Veröffentlichungstag der Patentschrift : **26.01.83**

(51) Int. Cl.³ : **C 07 D303/16**, C 07 D301/14

(21) Anmeldenummer : **79102856.6**

(22) Anmeldetag : **08.08.79**

(54) **Verfahren zur Herstellung von Glycidylestern cycloaliphatischer Polycarbonsäuren.**

(30) Priorität : **16.08.78 DE 2835881**

(43) Veröffentlichungstag der Anmeldung : **20.02.80 Patentblatt 80/04**

(45) Bekanntmachung des Hinweises auf die Patenterteilung : **26.01.83 Patentblatt 83/04**

(84) Benannte Vertragsstaaten : **CH DE FR GB IT NL**

(56) Entgegenhaltungen :
**DE A 1 942 557**
**DE A 2 602 776**
**DE B 1 082 263**
**DE B 1 083 797**
**FR A 1 269 628**

(73) Patentinhaber : **BAYER AG**
**Zentralbereich Patente, Marken und Lizenzen**
**D-5090 Leverkusen 1, Bayerwerk (DE)**

(72) Erfinder : **Rauleder, Gebhard, Dr.**
**Mozartstrasse 20**
**D-5657 Haan (DE)**
Erfinder : **Waldmann, Helmut, Dr.**
**Carl-Rumpff-Strasse 59**
**D-5090 Leverkusen (DE)**
Erfinder : **Bottenbruch, Ludwig, Dr.**
**Woehlerstrasse 5**
**D-4150 Krefeld 1 (DE)**
Erfinder : **Traenckner, Hans-Joachim, Dr.**
**Wedelstrasse 46**
**D-4150 Krefeld 1 (DE)**
Erfinder : **Vernaleken, Hugo, Dr.**
**Kreuzbergstrasse 147**
**D-4150 Krefeld 1 (DE)**
Erfinder : **Seifert, Hermann, Dr.**
**Ruwergasse 4**
**D-5000 Koeln 80 (DE)**
Erfinder : **Swodenk, Wolfgang, Dr.**
**Auf dem Broich 5**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 008 111 B1

# Verfahren zur Herstellung von Glycidylestern cycloaliphatischer Polycarbonsäuren

Die vorliegende Erfindung betrifft ein Verfahren zur Herstellung von Glycidylestern cycloaliphatischer Polycarbonsäuren aus Polyallylestern cycloaliphatischer Polycarbonsäuren.

Glycidylester cycloaliphatischer Polycarbonsäuren finden beispielsweise als Ausgangsprodukte zur Herstellung von Lacken und Kunststoffen, als Stabilisatoren, für die Herstellung von Gießharzen, zur Herstellung von Freiluftisolatoren, als Weichmacher und als Klebstoffe Verwendung (s. z.B. DE-B-1 082 263 und US-A-2 870 170).

Es ist bekannt, daß man Glycidylester aus Carbonsäuren, Carbonsäureanhydriden und Salzen von Carbonsäuren durch Umsetzung mit Epihalogenhydrin herstellen kann (s. z.B. DE-B-1 165 030, DE-A-1 643 777, US-A-2 448 602 und DE-A-2 023 148). Ein grundsätzlicher Nachteil dieser Verfahren besteht darin, daß salzhaltige umweltbelastende Abwässer gebildet werden.

In der GB-A-735 001 wird die Herstellung von Glycidylestern durch Umsetzen von Glycid mit Polycarbonsäurechloriden beschrieben. Dieses Verfahren hat den großen Nachteil, daß die Ausgangsmaterialien instabil und sehr reaktionsfähig sind, so daß bei der Herstellung, der Lagerung und der Verwendung dieser Verbindungen äußerste Vorsicht erforderlich ist (vgl. DE-A-2 032 148, S. 1, Z. 6).

Eine Möglichkeit, Olefine in Epoxide umzuwandeln unter Umgehung dieser Nachteile, besteht in der Anwendung der « Prileschajew-Reaktion » (vgl. N. Prileschajew, Ber. dtsch. chem. Ges. *42,* 4811, (1909). Bei dieser Reaktion handelt es sich um einen elektrophilen Angriff einer Percarbonsäure auf ein Olefin (vgl. K.D. Bingham, G.D. Meakins, G.H. Whitham, Chem. Commun. 1966, S. 445 und 446). Aus diesem Grunde nimmt die Reaktivität des Olefins mit fallender Nucleophilie der Doppelbindung ab. Deshalb erschweren elektronegative Substituenten die Epoxidation (vgl. S.N. Lewis in R.L. Augustin, « Oxidation » Vol. I, S. 227, Z. 9-13, Marcel Dekker, New York (1969)).

Elektronenziehende Gruppierungen wie z.B. Carboxyl, Carbonyl usw. vermindern die Reaktionsgeschwindigkeit enorm (vgl. H. Batzer und E. Nikles, Chimia, Vol. 16, Seite 62 (1962)). Insbesondere lassen sich daher Allylester nicht ohne weiteres mit Percarbonsäuren epoxidieren (vgl. DE-OS-2 023 148). Infolge der geringen Reaktionsfähigkeit ihrer Doppelbindung sind hohe Temperaturen und lange Reaktionszeiten erforderlich, was zur Bildung von unerwünschten Nebenprodukten, wie Dihydroxy- und Hydroxyacyloxy-Derivaten der Ausgangsprodukte, Anlaß gibt (vgl. S.N. Lewis in R.L. Augustin. « Oxidation » Vol. 1, S. 233, Z. 6-11, Marcel Dekker, New York (1969)).

In der GB-A-862 588 wird vorgeschlagen, den Diallylester der Terephthalsäure mit 50 %igem Wasserstoffperoxid in Gegenwart eines Kationenaustauschers, der Essigsäure adsorbiert enthält, zu epoxidieren (vgl. S. 2, Z. 15). Die Verwendung eines Kationenaustauschers als Katalysator hat jedoch den Nachteil, daß er vom Reaktionsgemisch angegriffen wird, so daß eine wiederholte Verwendung nur begrenzt möglich ist (vgl. DE-B-1 082 263, S. 1, Z. 40).

In der DE-B-1 082 263 wird im Beispiel 9, S. 8, Zeile 40, die Epoxidation von Terephthalsäurediallylester mit « in situ » hergestellter Peressigsäure in Gegenwart von Aluminiumoxid beschrieben. Für eine technische Anwendung stellt jedoch die große Menge an festem Katalysator im Reaktionsgemisch (70 g Aluminiumoxid auf 1 Mol Terephthalsäurediallylester) einen besonderen Nachteil dar. Der Katalysator muß nach beendeter Reaktion vom Reaktionsgemisch abgetrennt, gereinigt und regeneriert werden, wobei die Häufigkeit seiner Wiederverwendung sehr begrenzt ist.

In zwei weiteren Patentschriften (US-A-3 155 638 und FR-A-1 394 195) wird vorgeschlagen, zur Epoxidation von äthylenisch ungesättigten Carbonsäureestern Monoperphthalsäure zu verwenden. Ein Nachteil dieser Methode ist jedoch die technisch aufwendige Rückgewinnung der Phthalsäure aus dem Reaktionsgemisch. So wird in der US-A-3 155 638 im Beispiel 11, S. 8 und 9, nach der Umsetzung von Phthalsäurediallylester mit Phthalsäureanhydrid und Wasserstoffperoxid die abgeschiedene Phthalsäure abfiltriert. Der im Filtrat verbleibende Rest muß durch wiederholtes Waschen mit Wasser und Natronlauge rückgewonnen werden und daran anschließend müssen zur Wiedergewinnung der in den wäßrigen Phasen enthaltenden Phthalsäure diese wieder angesäuert werden, was zur Bildung von unerwünschten Salzabfällen führt.

Auch die Verwendung von m-Chlorperbenzoesäure zur Epoxidation der Allylester von Polycarbonsäuren, wie sie in der Literatur vorgeschlagen wurde (vgl. S.R. Sandler und F.R. Berg, J. Chem. und Eng. Data, Vol. 11, S. 447 und 448 (1966)), ist für eine technische Anwendung nicht geeignet, da die vorgeschlagenen Reaktionsbedingungen von 3 bis 5 °C, die Verwendung von kostspieliger Kühlsole notwendig macht und die angegebene Reaktionszeit von 3 Tagen technisch nicht akzeptabel ist. Trotz dieser aufwendigen Reaktionsbedingungen wurde beispielsweise Diglycidylterephthalat nur in einer Ausbeute von 28 % erhalten (vgl. Seite 448, Zeile 13).

In der US-A-2 761 870 wird in den Beispielen 1 bis 5 und Beispiel 8 die Epoxidierung von Crotylestern von Carbonsäuren mit 45 %iger Peressigsäure beschrieben. Auch hier sind die Reaktionsbedingungen, das Reaktionsgemisch muß 2 Tage bei 0° bis 25 °C stehen, für eine technische Anwendung (Verwendung von Kühlsole, lange Reaktionszeit) äußerst aufwendig. Nach der Reaktion wird die Essigsäure durch Waschen mit 20 %iger Natronlauge aus dem Reaktionsgemisch

entfernt. Will man die Essigsäure wiedergewinnen, muß die wäßrige Phase wieder angesäuert werden, was zur Bildung von umweltbelastenden Salzabfällen führt.

Bei den genannten Verfahren zur Herstellung von Oxiranen durch Umsetzung der entsprechenden Olefine mit Percarbonsäuren ist die Möglichkeit ihrer Anwendung zur Herstellung von Glycidylestern cycloaliphatischer Polycarbonsäuren nicht angegeben.

Aus der DE-B-10 83 797 ist ein Verfahren zur Herstellung von Epoxiden durch Umsetzung von Monoolefinen mit 7 bis 12 C-Atomen mit Peressigsäure bekannt, bei dem man das Reaktionsgemisch in Gegenwart eines Überschusses an Olefin destilliert, der ausreicht, um die entstandene Essigsäure als azeotropes Gemisch zu entfernen. Diese Destillation wird bei Temperaturen unter 100 °C durchgeführt, und man kann in Gegenwart eines über dem Siedepunkt des Epoxids siedenden Treibmittels arbeiten. Das erfindungsgemäße Verfahren unterscheidet sich von diesem Verfahren wesentlich. So entstehen erfindungsgemäß höher als Essigsäure siedende Carbonsäuren, von denen nicht bekannt ist, ob sie mit den erfindungsgemäß einzusetzenden Allylestern Azeotrope bilden können. Solche Azeotrope hätten in jedem Fall aber wesentlich höhere Siedepunkte als die gemäß der DE-B-1 083 797 auftretenden. Außerdem können die erfindungsgemäß einzusetzenden Allylester nicht im Überschuß eingesetzt werden, weil sonst die Bildung von Polyglycidylestern nicht möglich ist. Der erfindungsgemäß zuzufügende Zusatzstoff soll zwischen der entstehenden Carbonsäure und dem nächst höher siedenden Bestandteil sieden, nicht oberhalb des gebildeten Epoxids. Erfindungsgemäß kann die Destillation nicht auf Temperaturen unter 100 °C begrenzt werden. In der DE-B-1 083 797 wird dagegen ausgesagt, daß bereits über 50 °C die gebildete Carbonsäure Epoxide aufspalten kann, und erfindungsgemäß können Poly-Epoxide vorliegen, die erhöhte Angriffsmöglichkeiten haben im Vergleich zu den Monoepoxiden, die gemäß der DE-B-1 083 797 erhalten werden. Schließlicht geht aus dieser Druckschrift nicht hervor, daß das dort beschriebene Verfahren zur Epoxidation von mehrere epoxidierbare Gruppen enthaltenden Verbindungen geeignet ist, die bekanntermaßen schwierig zu epoxidieren sind. Aus allen diesen Gründen kann dieses bekannte Verfahren das erfindungsgemäße Verfahren nicht nahelegen.

Es wurde nun ein Verfahren zur Herstellung von Glycidylestern cycloaliphatischer Polycarbonsäuren gefunden, das dadurch gekennzeichnet ist, daß man Allylester cycloaliphatischer Polycarbonsäuren der allgemeinen Formel

$$R(-\overset{\overset{O}{\|}}{C}-O-CH_2-CH=CH_2)_n$$

worin

R einen cycloaliphatischen Rest, der substituiert sein kann, bedeutet und

n für 2, 3 oder 4 steht,

mit einer 3 bis 4 Kohlenstoffatome enthaltenden Percarbonsäure in Gegenwart von organischen Lösungsmitteln bei einer Temperatur von 30 bis 100 °C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem organischen Lösungsmittel durch Destillation abtrennt.

In der angegebenen Formel bedeutet R vorzugsweise einen cycloaliphatischen Ring mit 5 bis 12 C-Atomen. Besonders bevorzugt bedeutet R einen cycloaliphatischen Ring mit 6 C-Atomen. Der cycloaliphatische Ring R kann beispielsweise mit Alkyl-, Aryl-, Alkoxy- und/oder Halogengruppen substituiert sein, wobei die Kohlenstoff enthaltenden Gruppen beispielsweise 1 bis 10 C-Atome aufweisen können. Bevorzugte Substituenten sind Methyl-, Äthyl- und Methoxygruppen.

In der angegebenen Formel bedeutet n vorzugsweise 2.

Im einzelnen seien als Beispiele für cycloaliphatische Polyallyleser die Polyallylester von Hexahydrophthalsäure, Hexahydroisophthalsäure, Hexahydroterephtalsäure, 3-Methylhexahydrophthalsäure, 3,5-Dimethyl-hexahydrophthalsäure, 3-Methoxy-hexahydrophthalsäure und hydrierte Benzoltri- und -tetracarbonsäuren genannt.

Besonders geeignet zur Umsetzung mit Percarbonsäuren nach dem erfindungsgemäßen Verfahren sind die Diallylester von 3-Methyl-hexahydrophthalsäure, 3-Methoxyhexahydrophthalsäure, Hexahydrophthalsäure, Hexahydroisophthalsäure und Hexahydroterephthalsäure. Ganz besonders geeignet sind die Diallylester von Hexahydrophthalsäure, Hexahydroisophthalsäure und Hexahydroterephthalsäure.

Die nach dem erfindungsgemäßen Verfahren einsetzbaren Allylester cycloaliphatischen Polycarbonsäuren können in an sich bekannter Weise erhalten werden, beispielsweise durch Veresterung der entsprechenden Säureanhydride mit Allylalkohol.

Als organische Lösungsmittel können im erfindungsgemäßen Verfahren die verschiedensten unsubstituierten und substituierten Kohlenwasserstoffe verwendet werden, die unter Reaktionsbedingungen flüssig sind und unerwünschte Nebenreaktionen nicht oder nur in ganz untergeordnetem Maße eingehen. Als Kohlenwasserstoffe können z.B. verwendet werden aliphatische und cycloaliphatische Kohlenwasserstoffe wie Hexan, Heptan, Octan, 2-Äthyl-hexan, Decan, Dodecan, Cyclohexan, Methylcyclopentan und Petroläther, weiterhin aromatische Kohlenwasserstoffe wie Benzol, Nitrobenzol, Toluol, Äthylbenzol, Cumol, Diisopropylbenzol, Xylol und Chlorbenzol, weiterhin sauerstoffhaltige Kohlenwasserstoffe wie Diäthyläther, Diisopropyläther, Dibutyläther, Tetrahydrofuran, Dioxan, Essigsäureäthylester, Essigsäuremethylester, Essigsäurepropylester, Essigsäurebutylester, Propionsäuremethylester, Propionsäureäthylester, Propionsäurepropylester, Buttersäuremethylester, Buttersäureäthylester, Buttersäurepropylester, Buttersäurebutylester, Benzoesäuremethylester und Benzoesäureäthylester, weiterhin chlorierte Kohlenwasserstoffe wie Methylenchlorid, Chloroform, Tetrachlorkohlenstoff, 1-Chloräthan, 1,2-Dichloräthan, 1,1-Dichloräthan, 1,1,2,2-Tetrachloräthan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Bevorzugte Lösungsmittel sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Chlorbenzol, von den aliphatischen und cycloaliphatischen Kohlenwasserstoffen 2-Äthylhexan, Cyclohexan und Methylcyclopentan und von den sauerstoffhaltigen Kohlenwasserstoffen Tetrahydrofuran, Propionsäureäthylester und Benzoesäureäthylester.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Chlorbenzol, von den aliphatischen und cycloaliphatischen Kohlenwasserstoffen Cyclohexan und von den sauerstoffhaltigen Kohlenwasserstoffen Propionsäureäthylester und Benzoesäureäthylester.

Verwendet werden können auch Lösungsmittelgemische der verschiedenen oben angegebenen organischen Lösungsmittel.

Erfindungsgemäß verwendbare Percarbonsäuren sind Perpropionsäure, Perbuttersäure und Perisobuttersäure. Bevorzugt verwendet werden Perpropionsäure und Perisobuttersäure. Besonders bevorzugt ist Perpropionsäure. Diese Percarbonsäuren, gelöst in einem der genannten organischen Lösungsmittel, können z.B. nach dem in der DE-A-2 262 970 beschriebenen Verfahren hergestellt werden, bei dem wäßriges Wasserstoffperoxid mit der entsprechenden Carbonsäure in Gegenwart von Schwefelsäure umgesetzt und anschließend die entstandene Percarbonsäure mit einem organischen Lösungsmittel aus dem Reaktionsgemisch extrahiert wird.

Gegebenenfalls kann die so erhaltene Percarbonsäurelösung in dem organischen Lösungsmittel noch weiter gereinigt werden, insbesondere um den Gehalt an Wasser, Wasserstoffperoxid und Schwefelsäure zu erniedrigen.

Im allgemeinen verwendet man die Percarbonsäuren in Form einer Lösung in einem organischen Lösungsmittel. Der artige Percarbonsäurelösungen können beispielsweise 10 bis 30 Gew.-% der jeweiligen Percarbonsäure, bezogen auf die Lösung, enthalten. Die Allylester können als solche oder ebenfalls gelöst in einem oder mehreren der vorstehend genannten Lösungsmittel eingesetzt werden, wobei beliebig konzentrierte Lösungen der Allylester zum Einsatz gelangen können. Vorzugsweise werden die Allylester als solche eingesetzt und organische Lösungsmittel nur in Form der Percarbonsäurelösung zugegeben.

Das Molverhältnis von eingesetzter Percarbonsäure zu eingesetztem Polyallylester kann in weiten Grenzen schwanken. Beispielsweise kann dieses Molverhältnis 0,1 : 1 bis 10 : 1 betragen. Bevorzugt wird ein Molverhältnis von 0,15 : 1 bis 7 : 1 angewendet. Ganz besonders vorteilhaft ist es, ein Molverhältnis von 0,2 bis 5 Mol Persäure je Mol Polyallylester anzuwenden.

Der Wassergehalt der verwendeten Percarbonsäure soll im allgemeinen möglichst niedrig sein. Wassermengen bis 10 Gew.-% in der Percarbonsäurelösung sind im allgemeinen nicht störend. Geeignet ist beispielsweise eine Percarbonsäurelösung mit einem Wassergehalt von bis zu 2 Gew.-%. Vorzugsweise verwendet man eine Percarbonsäurelösung, die weniger als 1 Gew.-% Wasser enthält. Besonders bevorzugt ist ein Wassergehalt von weniger als 0,1 Gew.-%.

Der Wasserstoffperoxidgehalt der verwendeten Percarbonsäure in einem der oben genannten organischen Lösungsmittel soll im allgemeinen möglichst niedrig sein. Er kann beispielsweise bis zu 1 Gew.-%, bezogen auf die Percarbonsäurelösung, betragen. Vorteilhaft arbeitet man bei einem Gehalt von weniger als 0,5 Gew.-%. Besonders vorteilhaft ist es, die Umsetzung mit einer Percarbonsäurelösung durchzuführen, die einen Wasserstoffperoxidgehalt unterhalb 0,2 Gew.-% aufweist.

Der Mineralsäuregehalt der verwendeten Percarbonsäure soll möglichst niedrig sein. Vorteilhaft ist es, die Reaktion mit einer Percarbonsäurelösung durchzuführen, die einen Mineralsäuregehalt unterhalb 0,005 Gew.-% besitzt. Besonders vorteilhaft ist ein Mineralsäuregehalt von weniger als 0,001 Gew.-%.

Das erfindungsgemäße Verfahren wird in dem Temperaturbereich von 30-100 °C durchgeführt. Bevorzugt arbeitet man bei 40-80 °C, besonders bevorzugt bei 50-75 °C. In Sonderfällen können Verwendung eines Katalysators, wie z.B. eines die angegebenen Temperaturen auch unter- oder überschritten werden.

Neben der Arbeitsweise unter isothermen Bedingungen, d.h. Einhaltung einer einheitlichen Temperatur im gesamten Reaktionsgemisch,

kann man das erfindungsgemäße Verfahren auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das erfindungsgemäße Verfahren kann bei den verschiedensten Drucken durchgeführt werden. Im allgemeinen arbeitet man bei Normaldruck. Das Verfahren kann jedoch auch bei Unter- oder Überdruck durchfeführt werden.

Die Durchführung des erfindungsgemäßen Verfahrens kann diskontinuierlich oder kontinuierlich in den für Umsetzungen dieser Art üblichen Vorrichtungen, wie Rührwerkskessel, Siedereaktoren, Röhrenreaktoren, Schlaufenreaktoren oder Schleifenreaktoren, erfolgen.

Als Werkstoffe für die Reaktionsapparate zur Durchführung des erfindungsgemäßen Verfahrens können beispielsweise Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die Schwermetallionen durch Komplexbildung inaktivieren können. Bekannte Substanzen solcher Art sind beispielsweise Gluconsäure, Äthylendiamintetraessigsäure, Natriumsilicat, Natriumpyrophosphat, Natriumhexametaphosphat, Dinatriumdimethylpyrophosphat oder $Na_2$ (2-Äthyl-hexyl)$_5$($P_3O_{10}$)$_2$ (vgl. DE-AS 1 056 596, Spalte 4, Zeile 60 ff.).

Die Reaktionswärme kann durch innen- oder außenliegende Kühler abgeführt werden. Zur Ableitung der Reaktionswärme kann die Umsetzung auch unter Rückfluß (z.B. in Siedereaktoren) durchgeführt werden.

Der Allylester und die Percarbonsäure können auf beliebige Weise zusammengebracht werden. Beispielsweise kann man beide Komponenten gleichzeitig oder nacheinander in beliebiger Reihenfolge in das Reaktionsgefäß einbringen. Bei diskontinuierlicher Arbeitsweise wird vorzugsweise der Allylester vorgelegt und dann die Percarbonsäurelösung zugegeben. Die Reaktionstemperatur kann dabei vor oder nach der Zugabe der Percarbonsäure eingestellt werden. Man kann auch die beiden Komponenten bei Raumtemperatur gleichzeitig in das Reaktionsgefäß eingeben und dann die Reaktionstemperatur einstellen. Bei kontinuierlicher Arbeitsweise kann man die beiden Komponenten gemeinsam oder getrennt dem Reaktor zuführen. Bei Verwendung mehrerer Reaktoren, die beispielsweise als Kaskade hintereinander geschaltet sein können, kann es vorteilhaft sein, den Allylester nur in den ersten Reaktor einzubringen. Man kann die Allylesterzugabe jedoch auch auf mehrere Reaktoren verteilen.

Es kann weiterhin von Vorteil sein, die zur Erreichung des gewünschten Endproduktes (mono, partiell oder vollständig epoxidierte Polyallylester) benötigte Menge Percarbonsäure chargenweise zuzugeben. Dabei kann es von Vorteil sein, wenn das gewünschte Endprodukt partiell bzw. vollständig epoxidierter Polyallylester sein soll, den monoepoxidierten bzw. partiell epoxidierten Polyallylester aus dem Reaktionsgemisch abzutrennen und einer erneuten Umsetzung mit Percarbonsäure bis zum gewünschten Endprodukt zu unterwerfen. Bei der chargenweisen, Zugabe der Percarbonsäure kann es auch von Vorteil sein, nach Zugabe einzelner Chargen die aus der Percarbonsäure entstandene Carbonsäure aus dem Reaktionsgemisch zu entfernen, z.B. durch Extraktion mit Wasser oder durch Destillation.

Die nach Durchführung des erfindungsgemäßen Verfahrens erhaltenen Reaktionsgemische enthalten im allgemeinen das verwendete organische Lösungsmittel, die aus der Percarbonsäure entstehende Carbonsäure und epoxidierten Polyallylester, wobei gegebenenfalls partiell epoxidierter, monoepoxidierter und unumgesetzter Polyallylester sowie gegebenenfalls geringe Menge von hochsiedenden Nebenprodukten im Reaktionsgemisch vorhanden sein können.

Die Aufarbeitung des erhaltenen Reaktionsgemisches erfolgt durch Destillation. Dabei kann man so verfahren, daß man die einzelnen Komponenten in der Reihenfolge ihrer Siedepunkte abdestilliert. Dabei ist es von Vorteil, die Destillation im Vakuum und unter Verwendung von Verdampfern, die kurze Verweilzeiten und eine Verdampfung mit geringer thermischer Belastung des Reaktionsgemisches ermöglichen, durchzuführen. Erfindungsgemäß wird vor der destillativen Aufarbeitung dem Reaktionsgemisch ein geeigneter Zusatzstoff zugefügt, der zwischen der Carbonsäure und dem nächst höher siedenden Bestandteil des Reaktionsgemisches siedet. Dadurch erreicht man eine vollständige Abtrennung der Carbonsäure bei relativ niedrigen Sumpftemperaturen, was hinsichtlich der Minimierung der Bildung von Nebenprodukten aus der Carbonsäure und den epoxidierten Bestandteilen des Reaktionsgemisches von Vorteil ist. Erfindungsgemäß wird dann die Carbonsäure zusammen mit dem Lösungsmittel abgetrennt.

Es kann vorteilhaft sein, dem Reaktionsgemisch vor oder Während der destillativen Aufarbeitung, Stabilisatoren zuzusetzen, welche die Bildung von Hochsiedern und Polymerisaten verhindern.

Das erfindungsgemäße Verfahren hat eine Reihe von Vorteilen. Mit diesem Verfahren ist es erstmals möglich, nach der sogenannten Prileschajew-Reaktion in technischem Maßstab Glycidylester cycloaliphatischer Polycarbonsäuren herzustellen und dadurch die Nachteile des Epihalogen-hydrin-Verfahrens zu vermeiden.

Gegenüber den eingangs zitierten Anwendungen der Prileschajew-Reaktion besteht ein besonderer Vorteil des erfindungsgemäßen Verfahrens darin, daß die aus der Percarbonsäure entstehende Carbonsäure durch solche Maßnahmen aus dem Reaktionsgemisch entfernt werden kann, bei denen keine umweltbelastenden salzhaltigen Abwässer anfallen. Die

Verwendung eines Katalysators, wie z.B. eines Ionenaustauschers oder Aluminiumoxid, während der Reaktion, ist nicht notwendig. Nach dem erfindungsgemäßen Verfahrens ist es möglich, sowohl monoepoxidierte, partiell epoxidierte und/oder vollständig epoxidierte Polyallylester cycloaliphatischer Polycarbonsäuren in hoher Reinheit technisch herzustellen.

Das folgende Beispiel erläutert die Erfindung. Sämtliche Prozentangaben sind, soweit nichts anders gesagt wird, Gewichtsprozente.

Beispiel

Zu 1 260 g (5 Mol) Hexahydrophthalsäurediallylester wurden 2 547,17 g einer 21,2 %-igen benzolischen Perpropionsäurelösung (6 Mol), die einen Wassergehalt unter 0,1 %, einen Wasserstoffperoxidgehalt unter 0,2 % und einen Mineralsäuregehalt unter 0,001 % besaß, bei 60 °C unter Rühren zugegeben. Nach einer Reaktionszeit von 6 Stunden betrug der Persäureumsatz 99,1 % und die Epoxidausbeute, bezogen auf umgesetzten Diallylester, über 80 %.

Nach Zugabe von 800 g Benzoesäureäthylester wurde in einer mit einem Fallstromverdampfer betriebenen Füllkörperkolonne bei einem Druck von 200 mbar benzol und Propionsäure als Kopfprodukt abgetrennt ; sie wurden in einer weiteren Kolonne in die reinen Komponenten aufgetrennt.

Die Redestillation des Sumpfproduktes bei 20 mbar ergab reinen Benzoesäureäthylester als Kopfprodukt und ein Sumpfprodukt, das im wesentlichen aus Hexahydrophthalsäurediallylester, Hexahydrophthalsäureallylglycidylester und Hexahydrophthalsäurediglycidylester bestand und anschließend fraktioniert wurde. Bei einem Vakuum von 0,3 mbar erfolgte die Auftrennung in die Komponenten.

**Ansprüche**

1. Verfahren zur Herstellung von Glycidylestern cycloaliphatischer Polycarbonsäuren, dadurch gekennzeichnet, daß man Allylester cycloaliphatischer Polycarbonsäuren der allgemeinen Formel

$$R(-\overset{\overset{\displaystyle O}{\|}}{C}-O-CH_2-CH=CH_2)_n$$

worin

R einen cycloaliphatischen Rest, der substituiert sein kann, bedeutet und

n für 2, 3 oder 4 steht,

mit einer 3 bis 4 Kohlenstoffatome enthaltenden Percarbonsäure in Gegenwart von organischen Lösungsmitteln bei einer Temperatur von 30 bis 100 °C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem organischen Lösungsmittel durch Destillation abtrennt.

2. Verfahren nach Anspruch 1, dadurch gekennzeichnet, daß man Perpropionsäure einsetzt.

3. Verfahren nach Anspruch 1 und 2, dadurch gekennzeichnet, daß man die Umsetzung in Gegenwart von 1,2-Dichlorpropan, Tetrachlorkohlenstoff, Benzol, Chlorbenzol, Cyclohexan, Propionsäureäthylester und/oder Benzoesäureäthylester durchführt.

4. Verfahren nach Anspruch 1 bis 3, dadurch gekennzeichnet, daß man Percarbonsäure in Form einer Lösung in einem organischen Lösungsmittel verwendet, wobei die Lösung weniger als 10 Gew.-% Wasser, weniger als 1 Gew.-% Wasserstoffperoxid und weniger als 0,005 Gew.-% Mineralsäure enthält.

5. Verfahren nach Anspruch 1 bis 4, dadurch gekennzeichnet, daß man 0,1 bis 10 Mol Percarbonsäure pro Mol Allylester anwendet.

6. Verfahren nach Anspruch 1 bis 5, dadurch gekennzeichnet, daß man dem Reaktionsgemisch vor oder während der destillativen Aufarbeitung Stabilisatoren zusetzt, welche die Bildung von Hochsiedern und Polymerisaten verhindern.

**Claims**

1. Process for the preparation of glycidyl esters of cycloaliphatic polycarboxylic acids, characterised in that allyl esters of cycloaliphatic polycarboxylic acids of the general formula

$$R(-\overset{\overset{\displaystyle O}{\|}}{C}-O-CH_2-CH=CH_2)_n$$

wherein

R denotes a cycloaliphatic radical, which can be substituted, and

n represents 2, 3 or 4,

are reacted with a percarboxylic acid containing 3 to 4 carbon atoms in the presence of organic solvents at a temperature of 30 to 100 °C and then the resulting reaction mixture is worked up by distillation, the carboxylic acid formed from the percarboxylic acid being separated off by adding to the reaction mixture an additive which has a boiling point between that of the carboxylic acid forming from the percarboxylic acid and that of the constituent of the reaction mixture which has the next highest boiling point and separating off the carboxylic acid together with the organic solvent by distillation.

2. Process according to Claim 1, characterised in that perpropionic acid is employed.

3. Process according to Claim 1 and 2, characterised in that the reaction is carried out in the presence of 1,2-dichloro-propane, carbon

tetrachloride, benzene, chlorobenzene, cyclohexane, ethyl propionate and/or ethyl benzoate.

4. Process according to Claim 1 to 3, characterised in that the percarboxylic acid is used in the form of a solution in an organic solvent, the solution containing less than 10 % by weight of water, less than 1 % by weight of hydrogen peroxide and less than 0.005 % by weight of mineral acid.

5. Process according to Claim 1 to 4, characterised in that 0.1 to 10 mols of percarboxylic acid are used per mol of allyl ester.

6. Process according to Claim 1 to 5, characterised in that stabilisers which prevent the formation of high-boiling constituents and polymers are added to the reaction mixture before or during the working up by distillation.

**Revendications**

1. Procédé de production d'esters de glycidyle d'acides polycarboxyliques cycloaliphatiques, caractérisé en ce qu'on fait réagir des esters allyliques d'acides polycarboxyliques cycloaliphatiques de formule générale

$$R(-\overset{\overset{O}{\|}}{C}-O-CH_2-CH=CH_2)_n$$

dans laquelle

R est un reste cycloaliphatique qui peut être substitué et

n est égal à 2, 3 ou 4,

avec un acide percarboxylique contenant 3 à 4 atomes de carbone, en présence de solvants organiques, à une température de 30 à 100 °C, puis on traite par distillation le mélange réactionnel obtenu, et on sépare alors l'acide carboxylique produit à partir de l'acide percarboxylique, en ajoutant au mélange réactionnel un additif qui bout entre l'acide carboxylique produit à partir de l'acide percarboxylique et le composant du mélange réactionnel à point d'ébullition immédiatement supérieur, et on sépare l'acide carboxylique par distillation conjointement avec le solvant organique.

2. Procédé suivant la revendication 1, caractérisé en ce qu'on utilise l'acide perpropionique.

3. Procédé suivant les revendications 1 et 2, caractérisé en ce qu'on conduit la réaction en présence de 1,2-dichloropropane, de tétrachlorure de carbone, de benzène, de chlorobenzène, de cyclohexane, de propionate d'éthyle et/ou de benzoate d'éthyle.

4. Procédé suivant les revendications 1 à 3, caractérisé en ce qu'on utilise l'acide percarboxylique sous la forme d'une solution dans un solvant organique, cette solution contenant moins de 10 % en poids d'eau, moins de 1 % en poids de peroxyde d'hydrogène et moins de 0,005 % en poids d'un acide minéral.

5. Procédé suivant les revendications 1 à 4, caractérisé en ce qu'on utilise 0,1 à 10 moles d'acide percarboxylique par mole d'ester allylique.

6. Procédé suivant les revendications 1 à 5, caractérisé en ce qu'on ajoute au mélange réactionnel, avant ou pendant le traitement par distillation, des agents stabilisants qui empêchent la formation de composés de haut point d'ébullition et de produits de polymérisation.